# EUROPÄISCHE PATENTANMELDUNG

(11) **EP 2 620 165 A1**
(43) Veröffentlichungstag der Anmeldung: **31.07.2013**
(21) Anmeldenummer: 13000216.5
(22) Anmeldetag: 16.01.2013
(51) Int. Cl.: A61L 2/26, G08B 21/24, A61L 2/28

(54) **Behälter zur Schulung der Händedesinfektion**

(30) Priorität: 26.01.2012 DE 102012001475
(71) Anmelder: Bode Chemie GmbH, 22525 Hamburg (DE)
(72) Erfinder: Rhode, Katja, 20257 Hamburg (DE); Deuter, Joachim, 29640 Schneverdingen (DE); Barkmann, Christine, 22453 Hamburg (DE)

(57) **Zusammenfassung**

Die Erfindung betrifft einen Behälter (1) zur Prüfung der Verteilung von lumineszierenden Substanzen insbesondere auf der Handoberfläche eines Benutzers, mit einem Behälterunterteil (3) mit einem Behälterboden (5) und mit einem Behälteroberteil (2) mit einer Behälterdecke (6), wobei das Behälteroberteil (2) an dem Behälterunterteil (3) angelenkt ist und zwischen einer geschlossenen Position, in der es auf dem Behälterunterteil (3) aufliegt und dieses verschließt, und einer geöffneten Position, in der zwischen dem Behälterunterteil (3) und dem Behälteroberteil (2) zwei Hände eines Benutzers einführbar sind, verschwenkbar ist, wobei das Behälterunterteil (3) mindestens einen ersten Stauraum (18) umfasst, und wobei in dem Behälteroberteil (2) eine UV-Lichtquelle (13) aufgenommen ist, derart, dass die Lichtquelle in der geöffneten Position UV-Licht in Richtung auf das Behälterunterteil (3) emittieren kann, sobald die UV-Lichtquelle (13) eingeschaltet ist, wobei der Behälter (1) ein Öffnungsbegrenzungsmittel (7) aufweist, welches geeignet ist, den durch Behälterunterteil (3) und Behälteroberteil (2) in der geöffneten Position gebildeten Öffnungswinkel α zu begrenzen.

## Beschreibung

Die vorliegende Erfindung betrifft einen Behälter zur Prüfung der Verteilung von lumineszierenden Substanzen, insbesondere auf der Handoberfläche eines Benutzers.

Einer wirkungsvollen Händedesinfektion kommt insbesondere im medizinischen Bereich, vor allem in Krankenhäusern, Arztpraxen und Pflegeinstitutionen große Bedeutung zur. Unter Desinfektion wird dabei allgemein die effektive, irreversible Inaktivierung, Abtötung oder Entfernung von Mikroorganismen verstanden. Dabei zählen zu den Mikroorganismen Bakterien, Mykobakterien, Pilze, Hefen, Sporen, Prionen, Mykoplasmen und/oder Viren. Neben der Zusammensetzung des Desinfektionsmittels selbst kommt dem Vorgang der Anwendung des Mittels eine herausragende Bedeutung zu. Bei einem Händedesinfektionsmittel sind bei der Anwendung beispielsweise die Menge des Mittels, der Dosiervorgang, die Art und Dauer des Vorgang des Verreibens und Verteilens des Mittels auf der Handoberfläche und die Einwirkzeit zu berücksichtigen. Die Wirksamkeit der Handdesinfektion ist daher häufig unzureichend, sobald das medizinische Personal von den Anwendungsvorschriften abweicht und das Mittel nicht korrekt aufträgt. Es sind daher Schulungsvorrichtungen entwickelt worden, welche geeignet sind, dem Anwender die Qualität des Desinfektionsvorgangs zu demonstrieren und das korrekte Auftragen und Verteilen des Mittels zu trainieren.

So schlägt die DE202008012267U1 vor, dem Desinfektionsmittel eine fluoreszierende Substanz beizusetzen und die korrekte, ganzflächige Verteilung der Anwendung vermittels einer UV-Lampe sichtbar zu machen. Die UV-Lampe ist in ein Gehäuse aufgenommen, wobei das Gehäuse zwischen einer geschlossenen ersten Stellung und einer geöffneten zweiten Stellung umstellbar ist. In der geschlossen Stellung hat das Gehäuse die Form eines schmalen Kastens der Größe eines Aktenordners. Zur Öffnung des Gehäuses können mehrteilige Seitenwände, vorzugsweise auch der Boden des Gehäuses aufgeklappt werden. In der geöffneten zweiten Stellung nimmt das Gehäuse die Form eines größeren Quarders ein, welcher lediglich drei geschlossene Seitenwände aufweist, so dass das Gehäuse einen Zugang zum Einführen der Hände des Anwenders ermöglicht.

Es hat sich gezeigt, dass das Gehäuse in der geöffneten zweiten Stellung eine unbefriedigende Standfestigkeit aufweist und dazu neigt, nach vorn überzukippen, da die UV-Lampe in ein Elektronikgehäuse in einen oberen Bereich des Gehäuses aufgenommen ist und das Gewicht dieser Anordnung in der geöffneten zweiten Stellung aufgrund der ungünstigen Dimensionierungen, insbesondere aufgrund der geringen Bodenfläche des Gehäuses den Schwerpunkt der Vorrichtung weit nach oben und gleichzeitig nach außen verlagert. Konstruktionsbedingt lässt die geöffnete zweite Stellung des Gehäuses von oben außerdem zuviel Tageslichteintrag zu, welches das verlässliche Erkennen der fluoreszierenden Substanz unter Einwirkung des UV-Lichts erschwert.

Die vorliegende Erfindung löst diese Nachteile des Standes der Technik durch einen Behälter mit einem Behälterunterteil mit einem Behälterboden und mit einem Behälteroberteil mit einer Behälterdecke, wobei das Behälteroberteil an dem Behälterunterteil angelenkt ist und zwischen einer geschlossenen Position, in der es auf dem Behälterunterteil aufliegt und dieses verschließt, und einer geöffneten Position, in der zwischen dem Behälterunterteil und dem Behälteroberteil zwei Hände eines Benutzers einführbar sind, verschwenkbar ist, wobei das Behälterunterteil mindestens einen ersten Stauraum umfasst, und wobei in dem Behälteroberteil eine UV-Lichtquelle aufgenommen ist, derart, dass die Lichtquelle in der geöffneten Position UV-Licht in Richtung auf das Behälterunterteil emittieren kann, sobald die UV-Lichtquelle eingeschaltet ist, wobei der Behälter ein Öffnungsbegrenzungsmittel aufweist, welches geeignet ist, den durch Behälterunterteil und Behälteroberteil in der geöffneten Position gebildeten Öffnungswinkel zu begrenzen.

Dadurch, dass der Öffnungswinkel begrenzt ist, wird zum ersten sichergestellt, dass ausreichend Raum zum Einführen beider Hände eines Benutzers gegeben ist; zum zweiten ist der Eintrag von störendem Tageslicht reduziert; zum dritten ist die Standfestigkeit auch des geöffneten Behälters gewährleistet.

Vorzugsweise schließen sich an Behälterboden und Behälterdecke in der Z-Richtung, also senkrecht dazu, jeweils vier Seitenwände an: erste Längsseitenwände, deren Außenseiten in geschlossenem Zustand des Behälters die Rückseite des Behälters bilden; zweite Längsseitenwände, deren Außenseiten in geschlossenem Zustand die Vorderseite des Behälters bilden; erste Querseitenwände und zweite Querseitenwände, deren Außenseiten in geschlossenem Zustand die erste bzw. zweite Querseite des Behälters bilden.

In der geschlossenen Position ist vorzugsweise die Höhe H (Abmessung in Z-Richtung ZR) des Behälters geringer als die Länge L (Abmessung in Längsrichtung LR) und die Breite B (Abmessung in Querrichtung QR) des Behälters, was der Stabilität des Behälters zugute kommt. Besonders bevorzugt weisen das Behälteroberteil und das Behälterunterteil eine Länge L von 250-700 mm, insbesondere von 300-600 mm und eine Breite B von 100-400 mm, insbesondere von 150-300 mm auf. Vorzugsweise ist die Breite B geringer als die Länge L. Die Höhe H des Behälters, mithin gleichsam der Abstand zwischen den Außenseiten von Behälterboden und Behälterdecke beträgt in geschlossenem Zustand 30-200 mm, insbesondere von 40-100 mm.

Nach einer Weiterbildung der Erfindung beträgt der durch das Öffnungsbegrenzungsmittel begrenzte Öffnungswinkel 35-90 °, insbesondere 40-85°, weiter insbesondere 45-80°.

Bevorzugt umfasst das Öffnungsbegrenzungsmittel mindestens ein dem Fachmann an sich bekanntes Aufstellscharnier.

Weiter bevorzugt umfasst oder bildet das Aufstellscharnier außerdem ein Arretier- oder Haltemittel, welches dass Behälteroberteil gegen das Eigengewicht des Behälteroberteils in der geöffneten Position hält. In einer alternativen Ausführungsform kann das Arretier- oder Haltemittel auch als eine separate Komponente ausgebildet sein.

In einer bevorzugten Ausführungsform erstreckt sich die UV-Lichtquelle entlang der Längsrichtung LR. Bevorzugt ist die UV-Lichtquelle in Querrichtung QR von der ersten Längsseitenwand des Behälteroberteils weiter, insbesondere um zumindest 10% weiter, weiter insbesondere um zumindest 20% weiter beabstandet als von der zweiten Längsseitenwand des Behälteroberteils. Solchenfalls ist der Lichteinfall in der geöffneten Position vorteilhaft derart ausgerichtet, dass die in den Zwischenraum zwischen Behälterober- und -unterteil eingeführten Hände ausreichend beleuchtet sind. Außerdem ist diese Anordnung der UV-Lichtquelle der Stabilität des Behälters in der geöffneten Position zuträglich.

Um den Lichteinfall der UV-Lichtquelle auf den Innenraum des geöffneten Behälters zu richten und die Augen der Anwender vor der UV-Strahlung zu schützen, ist vorzugsweise vorgesehen, das Behälteroberteil mit einem erstes Blendschutzmittel zu versehen.

In den Ebenen, welche in der geöffneten Position seitlich zwischen Querseitenwänden von Behälterober- und -unterteil aufgespannt werden, sind nach einer Weiterbildung der Erfindung Verdunkelungsmittel angeordnet, welche vorzugsweise durch flexible, vorzugsweise textile Materialabschnitte gebildet sind, die einen störenden seitlichen Einfall von Tages- oder Raumlicht reduzieren helfen. In einer weiteren Weiterbildung der Erfindung kann der Behälter ein Bild- oder Filmaufnahmegerät umfassen oder es ist ein Bild- oder Filmaufnahmegerät an den Behälter anschließbar, so dass die Trainingsergebnisse dokumentiert werden können.

Der Behälter hat in der geschlossenen Position in etwa die Form und Größe eines Aktenkoffers. Weiter vorzugsweise weist der Behälter ein Anfassmittel, wie einen Henkel oder eine Grifflasche auf, so dass der Behälter im geschlossenen Zustand, vorzugsweise in der Form eines Aktenkoffers erfasst und getragen werden kann.

Bevorzugt umfasst das Behälterunterteil zumindest einen zweiten Stauraum, vorzugsweise weitere Stauräume.

Nach eine besonders bevorzugten Ausführungsform bildet der erste und/oder der zweite Stauraum des Behälterunterteils einen Raum zur Aufnahme verschiedener Utensilien, welche zur Durchführung einer Schulung der Händedesinfektion verwendet werden können, wie vorzugsweise kleinerer bereits mit Desinfektionsmitteln und/oder mit Trainingssubstanzen, insbesondere Trainingsflüssigkeiten gefüllter oder noch zu füllender Behältnisse, insbesondere Fläschchen oder einer Beschreibung oder Gebrauchsanweisung, insbesondere im Format DIN A5. Es könnten auch Substanzen in trockenem, entwässerten Zustand wie Tabletten vorgesehen sein, welche zum frischen Ansatz der Trainingsflüssigkeiten zu nutzen sind.

Vorzugsweise ist der Innenraum des Behälterunterteils durch senkrecht zum Behälterboden angeordnete Zwischenwände bedarfsgerecht zur Aufnahme ein oder mehrerer der beschriebenen Utensilien unterteilt.

Gegenstand der Erfindung ist somit außerdem ein Behälter wie vorstehend beschrieben, wobei in den ersten Stauraum ein Desinfektionsmittel und/oder ein mit Trainingssubstanzen, insbesondere Trainingsflüssigkeiten gefülltes oder noch zu füllendes Behältnis insbesondere ein Fläschchen oder eine Beschreibung oder eine Gebrauchsanweisung, insbesondere im Format DIN A5 aufgenommen ist.

Die Trainingssubstanzen umfassen vorzugsweise wässrige oder alkoholische Flüssigkeiten, denen fluoreszierende Substanzen beigefügt oder beizufügen sind. Lotionen, Gele oder Cremes, denen fluoreszierende Substanzen beigefügt oder beizufügen sind, sind ebenfalls denkbar. Die Trainingssubstanzen können vorteilhaft wirkstofffrei, das heißt ohne Zusatz der eigentlichen Wirkstoffkomponente des Desinfektionsmittels, dessen Anwendung zu trainieren ist, vorliegen. Alternativ kann den Trainingssubstanzen die Wirkstoffkomponente bereits zugesetzt sein.

Es erweist sich als vorteilhaft, wenn die Oberflächen der den Innenraum des Behälterunterteils und/oder des Behälteroberteils bildenden oder diese unterteilenden Bauteile, insbesondere die Innenseite des Behälterbodens gegenüber der Einwirkung alkoholischer Lösungsmittel unempfindlich insbesondere inert ausgebildet sind.

Nach einer weiteren bevorzugten Ausführungsform sind die zuvor genannten Oberflächen UV-lichtbeständig ausgebildet.

Weiter vorzugsweise weist der Behälter einen Schalter auf, vermittels dessen die Lichtquelle ein- und ausschaltbar ist.

Der Behälter besitzt nach einer weiteren vorteilhaften Ausführungsform eine Steckverbindung, vermittels derer die Lichtquelle mit einer Stromquelle verbindbar ist.

Die Böden und/oder Seitenwände von Behälterunterteil und/oder Behälteroberteil bildenden Materialien umfassen vorzugsweise Aluminiumbleche oder bestehen daraus. Kunststoffmaterialien oder eine Kombination aus Aluminium und Kunststoffmaterialien sind ebenfalls denkbar und vorteilhaft.

Nach einem weiteren die Erfindung weiter ausbildenden Gedanken wirken korrespondierende Seitenwände des Behälterunterteils und des Behälteroberteils im Bereich ihrer Kanten im geschlossenen Zustand des Behälters zusammen. Insbesondere vermögen die freien Kanten der Seitenwände im geschlossenen Zustand des Behälters dichtend aneinander zu liegen. Solchenfalls könnten die freien Kanten des Behälteroberteils und/oder des Behälterunterteils mit einem Dichtungsmittel, beispielsweise einem Gummiprofil versehen sein.

Nach einer bevorzugten Ausführungsform weist die Außenseite des Bodens des Behälterunterteils Rutschverhinderungsmittel wie vorzugsweise flach bauende Gummifüßchen auf. Solchenfalls können mindestens zwei, vorzugsweise mindestens drei der Behälter in geschlossenem Zustand übereinander gestapelt werden.

Nach einer weiteren vorteilhaften Ausführungsform der Erfindung umfasst der Behälter Verschlussmittel, vermittels derer die geschlossene Position lösbar verriegelbar ist.

Nach einem weiteren Erfindungsgedanken wird der Behälter zur Schulung der korrekten Anwendung von Händedesinfektionsmitteln verwendet. Denkbar und vorteilhaft wäre auch, den Behälter zur Begutachtung von an anderen Körperteilen aufgetragenen Desinfektions- oder Trainingslösungen zu verwenden, insbesondere zu Schulungszwecken. Nach einem weiteren Erfindungsgedanken wird der Behälter verwendet, um die Verteilung von Desinfektions- oder Trainingslösungen auf medizinischen Gegenständen wie Instrumenten zu begutachten.

Nachstehend werden Ausführungsformen erfindungsgemäßer Behälter anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst der erfindungsgemäße Behälter auch Kombinationen der Einzelmerkmale der alternativen Formen. Es zeigen:
Figur 1, eine perspektivische Ansicht eines erfindungsgemäßen Behälters in der geöffneten Position
Figur 2, eine perspektivische Ansicht eines erfindungsgemäßen Behälters in der geschlossenen Position
Figur 3, perspektivische Ansicht eines weiteren erfindungsgemäßen Behälter in der geöffneten Position

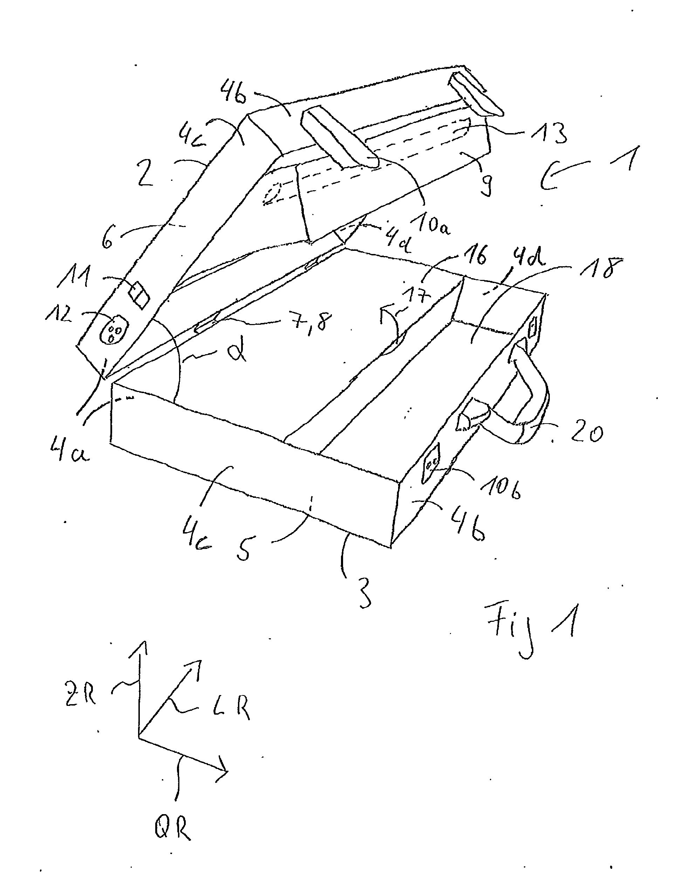

Figur 1 zeigt schematisch einen insgesamt mit dem Bezugszeichen 1 bezeichneten Behälter zur Prüfung der Verteilung von lumineszierenden Substanzen, insbesondere auf der Handoberfläche eines Benutzers. Der Behälter 1 ist nach Art eines Aktenkoffers ausgebildet und umfasst ein Behälteroberteil 2 mit einer Behälterdecke 6 und ein Behälterunterteil 3 mit einem Behälterboden 5. An Behälterboden 5 und Behälterdecke 6 schließen sich senkrecht dazu Seitenwände, nämlich erste Längsseitenwände 4a, deren Außenseiten in geschlossenem Zustand die Rückseite des Behälters bilden, zweite Längsseitenwände 4b, deren Außenseiten in geschlossenem Zustand die Vorderseite des Behälters bilden, erste Querseitenwände 4c, und zweite Querseitenwände 4d, deren Außenseiten in geschlossenem Zustand die erste bzw. zweite Querseite des Behälters bilden. Mit einer ersten Längsseitenwand 4a des Behälteroberteils 2 ist das Behälteroberteil 2 über Öffnungsbegrenzungsmittel 7 bildende Aufstellscharniere 8 an das Behälterunterteil 3 angelenkt.

Denkbar und vorteilhaft wäre außerdem, ein Scharnier mit einem Arretier- oder Haltemittel zu versehen, welches dass Behälteroberteil gegen das Eigengewicht des Behälteroberteils in der geöffneten Position hält. In einer alternativen Ausführungsform kann das Arretier- oder Haltemittel auch als eine separate Komponente ausgebildet sein. Solchenfalls kann das Arretier- oder Haltemittel auch an den jeweils korrespondierenden ersten Querseitenwänden 4c und/oder zweiten Querseitenwänden 4b von Behälterober- und -unterteil angeordnet sein

Das Behälterunterteil 3 umfasst einen ersten Stauraum 18 und einen zweiten Stauraum. Der zweite Stauraum befindet sich unterhalb eines Deckels 16, welcher in Richtung des Pfeils 17 hochschwenkbar ist, um den zweiten Stauraum freizugeben. Die erste und/oder der zweite Stauraum kann dazu vorbereitet sein zur Durchführung einer Schulung der Händedesinfektion geeignete Utensilien vorzuhalten und mit dem Behälter mitzuführen, wie vorzugsweise ein bereits mit Trainingslösung gefülltes oder noch zu füllendes Fläschchen oder einer Beschreibung oder eine Gebrauchsanweisung, insbesondere im Format DIN A5.

Angedeutet ist schematisch eine UV-Lichtquelle 13 in Form einer sich in der Längsrichtung LR erstreckenden UV-Röhrenlampe, welche in Querrichtung QR von der ersten Längsseitenwand 4a des Behälteroberteils weiter beabstandet ist als von einer zweiten Längsseitenwand 4b des Behälteroberteils. Die UV-Lichtquelle 13 ist vermittels in Figur 1 nicht dargestellter Halterungen und elektronischer Komponenten an das Behälteroberteil festgelegt und dazu vorbereitet über einen Schalter 11 an- und ausschaltbar zu sein. Der Behälter 1 kann eine Stromversorgungsquelle wie eine Batterie umfassen und/oder vermittels einer Steckverbindung 12 mit einer externen Stromquelle verbindbar sein. Die UV-Lichtquelle 13 ist in der Ansicht der Figur 1 hinter einem ersten Blendschutzmittel 9 verborgen. Das erste Blendschutzmittel 9 schützt das Auge des Benutzers vor dem UV-Licht und besitzt vorzugsweise auf seiner Innenseite lichtreflektierende Materialien, die das Licht in Richtung auf den Innenraum des geöffneten Behälters 1, also auf dem offenen Raum richtet, welcher durch Behälterober- und -unterteil 2, 3 aufgespannt ist. Um die UV-Lichtquelle 13 mechanisch zu schützen, könnte die Lampe in einem vorzugsweise allseitig geschlossenen Gehäuse angeordnet sein, welches eine transparente Kunststoffplatte oder ein Siebblech oder Ähnliches umfasst (nicht dargestellt).

Das Öffnungsbegrenzungsmittel 8 begrenzt den durch Behälterober- und -unterteil 2, 3 gebildeten Öffnungswinkel α auf etwa 70 ° (Figur 1).

Nachdem ein Benutzer den Vorgang der Händedesinfektion mit einem vorzugsweise alkoholischen Desinfektionsmittel oder einer vorzugsweise alkoholischen Trainingslösung vollzogen hat, welche mit einer lumineszierenden, insbesondere fluoreszierenden Substanz versehen ist, führt der Benutzer die Hände in den Innenraum des geöffneten Behälters 1 ein. Das auf die Handoberfläche fallende UV-Licht lässt dann die Verteilung der fluoreszierenden Substanz und damit die Gleichmäßigkeit der Verteilung des Desinfektions- oder Trainingsmittels erkennen.

Vermittels der miteinander korrespondierenden Verschlussmittel 10a, 10b, kann der Behälter in einer geschlossenen Position lösbar verriegelt werden und ist anschließend an einem Tragegriff 20 ergreifbar.

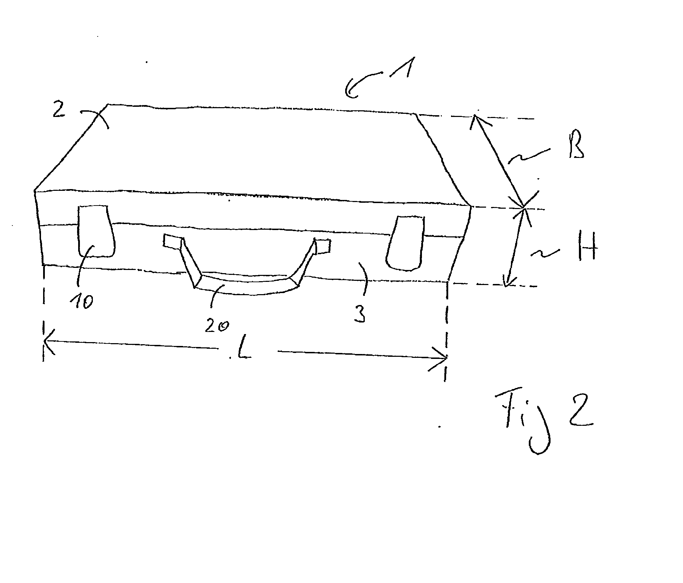

Figur 2 zeigt den Behälter 1 in der geschlossenen Position. Identische Bezugszeichen kennzeichnen die der Figur 1 entsprechenden Komponenten des Behälters 1. Die Länge L des Behälters 1 beträgt im dargestellten Fall 400 mm. Die Breite B beträgt 200 mm. Die Höhe H beträgt 55 mm.

Die komfortable Stellfläche des Behälters 1 (Länge L x Breite B) und die Begrenzung des Öffnungswinkels α stellen eine hohe Standfestigkeit des Behälters in der geöffneten Position sicher. Außerdem ist gewährleistet, dass vergleichsweise wenig störendes Tages- bzw. Raumlicht in den Innenraum des Behälters 1 fällt. Die Umgebung, in der das Training stattfindet, muss daher nicht abgedunkelt werden.

Die Böden und Seitenwände von Behälterunter- und -oberteil sind im dargestellten fall aus Aluminiumblechen gebildet. Denkbar wären auch Kunststoffmaterialien, massives Holz, Holzwerkstoffe oder andere üblicherweise behälterbildende Materialien, insbesondere auch Kombinationen hiervon zu verwenden.

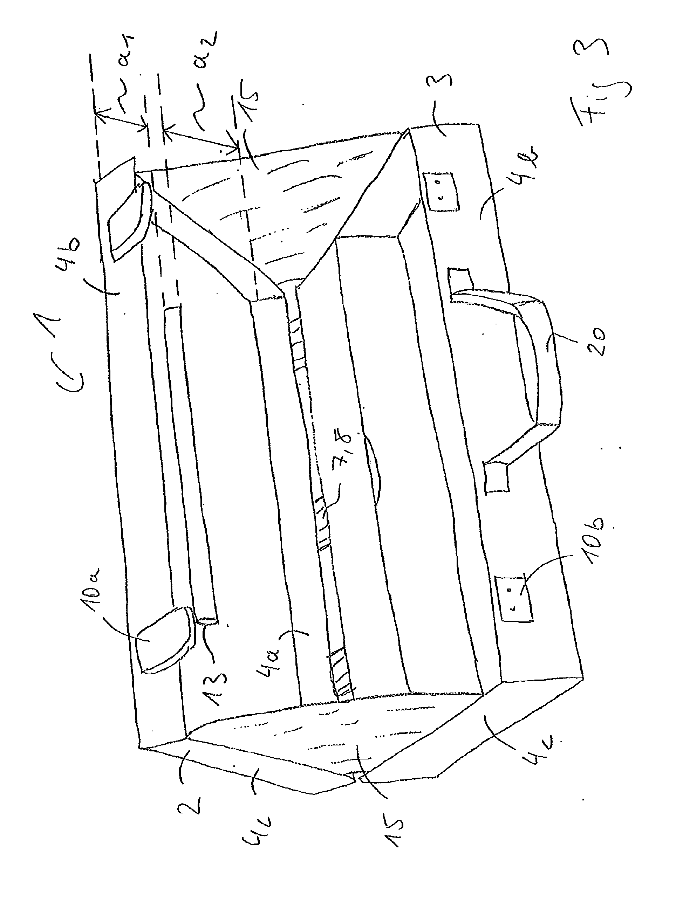

Figur 3 zeigt eine weitere Ausführungsform eines erfindungsgemäßen Behälters 1. Identische Bezugszeichen kennzeichnen wiederum die der Figur 1 entsprechenden Komponenten des Behälters 1. Die UV-Lichtquelle 13 ist in das Behälteroberteil 2 aufgenommen. In den Ebenen, welche in der geöffneten Position zwischen den jeweils korrespondierenden Querseitenwänden 4c, 4d von Behälterober- und - unterteil aufgespannt werden, sind Verdunkelungsmittel 15 angeordnet, welche durch flexible, vorzugsweise textile Materialabschnitte gebildet sind, die einen störenden seitlichen Einfall von Tages- oder Raumlicht reduzieren helfen. Erste Blendschutzmittel 9 wie in Figur 1 dargestellt könnten außerdem vorteilhaft vorgesehen sein. Der Abstand (kürzeste Distanz) a1 der UV-Röhrenlampe von der ersten Längsseitenwand 4a des Behälteroberteils 2 beträgt 10 cm. Der Abstand a1 der UV-Röhrenlampe von der zweiten Längsseitenwand 4b des Behälteroberteils beträgt 7 cm.

Es wäre denkbar und vorteilhaft, dass die Verdunkelungsmittel 15 nach einer alternativen Ausführungsform außerdem als Öffnungsbegrenzungsmittel fungieren. Solchenfalls könnten die Scharniere, welche das Behälteroberteil 2 an das Behälterunterteil 3 anlenken, ohne Öffnungsbegrenzungsmittel ausgeführt sein.

## Patentansprüche

1. Behälter (1) zur Prüfung der Verteilung von lumineszierenden Substanzen insbesondere auf der Handoberfläche eines Benutzers, mit einem Behälterunterteil (3) mit einem Behälterboden (5) und mit einem Behälteroberteil (2) mit einer Behälterdecke (6), wobei das Behälteroberteil (2) an dem Behälterunterteil (3) angelenkt ist und zwischen einer geschlossenen Position, in der es auf dem Behälterunterteil (3) aufliegt und dieses verschließt, und einer geöffneten Position, in der zwischen dem Behälterunterteil (3) und dem Behälteroberteil (2) zwei Hände eines Benutzers einführbar sind, verschwenkbar ist, wobei das Behälterunterteil (3) mindestens einen ersten Stauraum (18) umfasst, und wobei in dem Behälteroberteil (2) eine UV-Lichtquelle (13) aufgenommen ist, derart, dass die Lichtquelle in der geöffneten Position UV-Licht in Richtung auf das Behälterunterteil (3) emittieren kann, sobald die UV-Lichtquelle (13) eingeschaltet ist, wobei der Behälter (1) ein Öffnungsbegrenzungsmittel (7) aufweist, welches geeignet ist, den durch Behälterunterteil (3) und Behälteroberteil (2) in der geöffneten Position gebildeten Öffnungswinkel α zu begrenzen.

2. Behälter (1) nach Anspruch 1, **dadurch gekennzeichnet, dass** das Öffnungsbegrenzungsmittel (7) mindestens ein Aufstellscharnier (8) umfasst.

3. Behälter (1) nach einem der vorstehenden Ansprüche, **dadurch gekennzeichnet, dass** der Öffnungswinkel α 35-90 °, insbesondere 40-85°, weiter insbesondere 45-80° beträgt.

4. Behälter (1) nach einem der vorstehenden Ansprüche, **dadurch gekennzeichnet, dass** das Behälteroberteil (2) ein erstes Blendschutzmittel (9) umfasst.

5. Behälter (1) nach einem der vorstehenden Ansprüche, **dadurch gekennzeichnet, dass** der Behälter (1) ein Bildaufnahmegerät umfasst oder der Behälter (1) an ein Bildaufnahmegerät anschließbar ist.

6. Behälter (1) nach einem der vorstehenden Ansprüche, **dadurch gekennzeichnet, dass** das Behälteroberteil (2) und das Behälterunterteil (3) eine Länge L von 250-700 mm, insbesondere von 300-600 mm und eine Breite B von 100-400, insbesondere von 150-300 mm aufweisen.

7. Behälter (1) nach einem der vorstehenden Ansprüche, **dadurch gekennzeichnet, dass** der Behälter (1) in geschlossenem Zustand eine Höhe H von 30-200 mm, insbesondere von 40-100 mm aufweist.

8. Behälter (1) nach einem der vorstehenden Ansprüche, **dadurch gekennzeichnet, dass** der Behälter (1) ein Arretier- oder Haltemittel umfasst, welches dass Behälteroberteil (2) gegen das Eigengewicht des Behälteroberteils (2) in der geöffneten Position hält.

9. Behälter (1) nach einem der vorstehenden Ansprüche, **dadurch gekennzeichnet, dass** die Oberflächen der den Innenraum des Behälterunterteils (3) bildenden oder diesen unterteilenden Bauteile gegenüber der Einwirkung alkoholischer Lösungsmittel unempfindlich insbesondere inert ausgebildet sind.

10. Behälter (1) nach einem der vorstehenden Ansprüche, **dadurch gekennzeichnet, dass** Behälteroberteil (2) und Behälterunterteil (3) jeweils einen Boden (5, 6) sowie vier Seitenwände (4a, 4b, 4c, 4d) umfassen und dass die korrespondierenden Seitenwände (4a, 4b, 4c, 4d) des Behälterunterteils (3) und des Behälteroberteils (2) im Bereich ihrer Kanten im geschlossenen Zustand des Behälters (1) zusammenwirken.

11. Behälter (1) nach einem der vorstehenden Ansprüche, **dadurch gekennzeichnet, dass** die Außenseite des Bodens (5) des Behälterunterteils (3) Rutschverhinderungsmittel aufweisen, so dass mindestens zwei, vorzugsweise mindestens drei der Behälter (1) in geschlossenem Zustand übereinander stapelbar sind.

12. Behälter (1) nach einem der vorstehenden Ansprüche, **dadurch gekennzeichnet, dass** das Behälterunterteil (3) einen zweiten Stauraum umfasst.

13. Behälter (1) nach einem der vorstehenden Ansprüche, **dadurch gekennzeichnet, dass** der erste Stauraum (18) und/oder der zweite Stauraum einen Raum zur Aufnahme verschiedener Utensilien, welche zur Durchführung einer Schulung der Händedesinfektion verwendet werden können, wie vorzugsweise kleinerer bereits mit Desinfektionsmitteln und/oder mit Trainingssubstanzen, insbesondere Trainingsflüssigkeiten gefüllter oder noch zu füllender Behältnisse, insbesondere Fläschchen oder einer Beschreibung oder Gebrauchsanweisung, insbesondere im Format DIN A5 umfasst.

14. Behälter (1) nach einem der vorstehenden Ansprüche, **dadurch gekennzeichnet, dass** in den ersten Stauraum (18) und/oder in den zweiten Stauraum Desinfektionsmitteln und/oder mit Trainingssubstanzen, insbesondere Trainingsflüssigkeiten gefüllte oder noch zu füllende Behältnisse insbesondere Fläschchen oder einer Beschreibung oder eine Gebrauchsanweisung, insbesondere im Format DIN A5 aufgenommen ist oder sind.

15. Verwendung des Behälters (1) nach einem der vorstehenden Ansprüche zur Schulung der Anwendung von Händedesinfektionsmitteln.
